(19) Europäisches Patentamt
European Patent Office
Office européen des brevets

(11) Publication number: 0 022 816
B1

(12) EUROPEAN PATENT SPECIFICATION

(45) Date of publication of patent specification: 13.07.83

(51) Int. Cl.³: C 07 C 102/06

(21) Application number: 80900197.7

(22) Date of filing: 17.12.79

(86) International application number:
PCT/US79/01108

(87) International publication number:
WO 80/01564 07.08.80 Gazette 80/18

# (54) THE PREPARATION OF ANTIOXIDANT AMIDES.

(30) Priority: 26.01.79 US 6818

(43) Date of publication of application:
28.01.81 Bulletin 81/4

(45) Publication of the grant of the patent:
13.07.83 Bulletin 83/28

(84) Designated Contracting States:
DE FR GB

(56) References cited:
US - A - 3 462 486
US - A - 3 907 893
US - A - 4 105 693

JOURNAL OF ORGANIC CHEMISTRY, vol. 28, October 1963 Washington D.C. USA
R. J. DE FEO et al. "An improved method of synthesis of secondary amides from carboxylic esters", pages 2915—2917

(73) Proprietor: THE GOODYEAR TIRE & RUBBER COMPANY
1144 East Market Street
Akron, Ohio 44316 (US)

(72) Inventor: SHAH, Niranjan V.
1923 Kingsdale Drive
Stow, OH 44224 (US)

(74) Representative: Weyland, Joseph Jean Pierre
Goodyear International Tire Technical Center
Patent Department Avenue Gordon Smith
L-7750 Colmar-Berg (LU)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 022 816 B1

Courier Press, Leamington Spa, England

# The preparation of antioxidant amides

*Technical Field*

This invention pertains to aryl amides, in particular to an improved process for synthesizing N-(4-anilino phenyl) Amides and related compounds.

*Background*

US Patent 3,907,893 (referred to herein after as the Parker Patent) describes the basic process for synthesizing aromatic antioxidant amides, an improvement of which is the subject of this application. Additionally, two comparative samples of N-(4-anilinophenyl)methacrylamide designated samples 1 and 2 in column 7 of US 3,907,893 were prepared without isolation of the intermediate metallic salt by hydrolysis (conditions unspecified) of the entire solution containing the salt. These samples contained impurities which stopped polymerisation of butadiene/acrylonitrile.

US Patent 3,538,159 describes the use of alkali metal alkoxides as catalysts in the production of di-N-alkylamides.

The basic process comprises reacting an aliphatic or aromatic ester, either saturated or unsaturated, having formula (A)

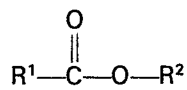
(A)

with an aromatic amine having the general formula (B)

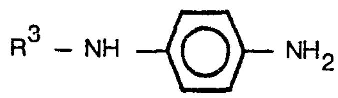
(B)

in the presence of (C), a base selected from the group consisting of alkali metal amides and alkali metal alkoxides to yield an intermediate metallic salt and an alcohol.

$R^1$ is selected from the group consisting of alkyl radicals having from one to 20 carbon atoms, aryl radicals having from six to 12 carbon atoms, cycloalkyl radicals having from five to 12 carbon atoms, alkenyl radicals having from two to 10 carbon atoms, $R^2$ is selected from the group consisting of alkyl radicals having from one to 10 carbon atoms, and alkenyl radicals having from two to 10 carbon atoms and $R^3$ is selected from the group consisting of alkyl radicals having from one to 20 carbon atoms, cycloalkyl radicals having from five to 12 carbon atoms, aryl radicals having from 6 to 12 carbon atoms and aralkyl radicals having from seven to 13 carbon atoms.

This reaction is performed in an aromatic solvent. The conversion in this first reaction is not complete, ranging generally from 50 to 90%. Because of this and because the base and the aromatic amine are sometimes charged in excess of the stoichiometric amount, the reaction product contains significant quantities of unreacted base and unreacted amine.

The next step in the Parker process is removal of the intermediate metal salt from the reaction mixture by some physical separation means, such as filtration. The excess of raw materials referred to above makes this separation difficult and time consuming because the particles in the reaction slurry are very fine (some being less than one micron in diameter). Alkali metal alkoxides, such as sodium methoxide, have the appearance of talcum powder, and thus the very small particle size of this material contributes to the filtration problem. The reaction slurry has the appearance of a milk shake, and the filter cake is a slimy material which tends to blind the filter medium. The filter cake is invariably saturated with solvent, and removal from the filter for eventual hydrolysis, becomes a very messy operation.

In the basic process, the filter cake is next washed with an organic solvent such as xylene to remove unreacted aromatic amine (the presence of which must be minimized in the final product). This may be followed by subsequent washes with the same or another solvent such as hexane or toluene. The filter cake is then reslurried in dilute, aqueous acid which hydrolyzes the metallic salt to the desired product (D).

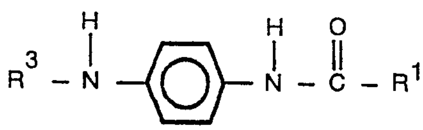
(D)

Prior to hydrolysis the filter cake may be dried. The solid product is then removed by filtration or another appropriate unit operation and is dried.

Some reactions yield intermediate metallic salts which are liquids, not susceptible to filtration but which can be separated from the solvent by extraction and/or decantation. The Parker process is applicable, with variation of separation techniques, to these various intermediates.

Because of the poor quality of the first filter cake, the particle size of the product upon hydrolysis is often very small, making the final filtration also difficult.

It is therefore highly desirable to alleviate the problems associated with the hydrolysis and filtration steps.

An article by DeFeoand and Strickler, "An Improved Method of Synthesis of Secondary Amides from Carboxylic Esters" in *J.O.C.*, Volume 28 p. 2915—2917 (1963), discusses the synthesis of amides by reaction between an ester and an amine using sodium methoxide. This article teaches the hydrolysis of the reaction mixture in dilute mineral acid (10% HCl used in example).

U.S. Patent 3,462,486 describes a method of preparing anilides of lower cycloalkanoic acids by reacting a lower alkyl ester of such acids with a primary aromatic amine and an alkali metal alcoholate distilling off the alcohol and neutralizing the reaction mixture with an aqueous acid.

*Summary of the Invention*

The Parker or ester process has been improved by replacing three of the finishing steps (1. removing the alkali metal salt of the first reaction by filtration or other means, 2. washing the salt (filter cake) with an organic solvent, and 3. hydrolyzing the salt with dilute acid). These three steps are replaced by the single step of hydrolyzing the salt in-situ by mixing the reaction product with water at a temperature at which most of the reaction product is suspended in the water phase and most of the unreacted aromatic amine is dissolved in the reaction solvent phase.

By following this technique, a number of advantages are realized:

1. When sodium methoxide is used for the base, it is transferred to the aqueous phase where it reacts with water forming sodium hydroxide and methanol, thus alleviating the fine "talcum powder" solid phase which caused problems during filtration, since sodium hydroxide and methanol are miscible with water.

2. Both the first filtration step and the accompanying wash step are eliminated.

3. The slurry resulting after hydrolysis is easy to filter and relatively free of organic solvent when water washed and air dried.

4. Decreased processing time has been realized through increased rates of hydrolysis and filtration.

5. A raw material saving is realized in the elimination of the organic solvent used in washing the filter cake.

6. Another advantage realized through this process improvement has been a decrease in water pollution. In the old process, there is not complete recovery of the reaction solvent as filtrate in the first filtration because a significant quantity of solvent remains with the filter cake. In the filtration following hydrolysis with the dilute acid, this residual reaction solvent is transferred to the aqueous filtrate and wash water, being a pollutant therein. On the other hand, because of the improved filtration achieved through this improvement, very little reaction solvent remains on the filter cake which is more in the nature of granules or pellets rather than fine particles or slime. Almost all of the reaction solvent is then recovered by a simple decantation of the filtrate, leaving very little in the aqueous phase.

The slurry resulting after in-situ hydrolysis does not have the slimy appearance of the old reaction slurry. This could be due to the increased particle size of the product over that which formerly occurred. It also could be due to the absence of the "talcum powder" unreacted base. This results in a much improved filtration rate and also gives a cake which is easier to wash with water.

The in-situ hydrolysis has been carried out with water alone and with water plus one or two organic solvents, such as hexane and xylene. When hexene or hexane and xylene were used together with water, an increase in the crude yield of the product was observed. It is believed that impurities (side reaction products) which would normally remain in the organic phase, were transferred to the aqueous phase when hexane was added to the solvent system. Although these side products added to the apparent or crude yield, they did not increase the true yield of N-(4-anilinophenyl)-amide. In fact, the amide produced by solvent-aided in-situ hydrolysis was somewhat lower in purity and performance than those made by in situ hydrolysis with water only. However, there is also disclosed an improved process for the synthesizing of an antioxidant amide comprising:

I. reacting, in an aromatic solvent, an aliphatic or aromatic ester, either saturated or unsaturated, having the formula:

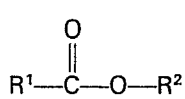

(A)

with an aromatic amine having the general formula

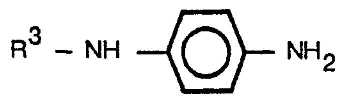

(B)

in the presence of (C) a base selected from the group consisting of alkali metal amides and alkali metal alkoxides to yield an intermediate metallic salt:

II. hydrolyzing the salt in situ by mixing the reaction product from step I with water at a temperature at which most of the reaction product is suspended in the water phase and most of the unreacted aromatic amine is dissolved in the reaction solvent phase to form an antioxidant amide of the general formula:

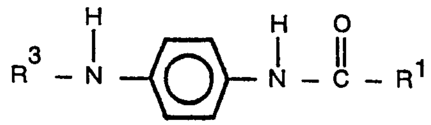

(D)

wherein $R^1$ is selected from the group consisting of alkyl radicals having from 1 to 20 carbon atoms, aryl radicals having from 6 to 12 carbon atoms, cycloalkyl radicals having from 5 to 12 carbon atoms, alkenyl radicals having from 2 to 10 carbon atoms, $R^2$ is selected from the group consisting of alkyl radicals having from 1 to 10 carbon atoms, and alkenyl radicals having from 2 to 10 carbon atoms, and wherein $R^3$ is selected from the group consisting of alkyl radicals having from 1 to 20 carbon atoms, cycloalkyl radicals having from 5 to 12 carbon atoms, aryl radicals having from 6 to 12 carbon atoms, and aralkyl radicals having from 7 to 13 carbon atoms.

The amides (D) described herein are useful as antioxidants in elastomers. They can be incorporated into elastomers by conventional techniques well known to those in the art, such as by addition to polymer latices or to solid polymers in an internal mixer or on a mill.

Amides formed from an unsaturated ester such as methyl methacrylate are copolymerizable with conventional unsaturated monomers such as those used in the preparation of unsaturated synthetic elastomers. When copolymerized with these monomers, the amide products become a part of the elastomer molecules, are non-volatile and are not extractable. These built-in antioxidants are advantageous for long term resistance in polymers. An amide produced by in-situ hydrolysis has been successfully copolymerized.

*Description of the Preferred Embodiments*

In-situ hydrolysis with water only, gives the best product with the highest purity, and the best polymerization rate and conversion for the copolymerizable amides. The use of water only has another advantage over the solvent-aided hydrolysis. After filtration of the hydrolyzed reaction product, the aqueous phase of the filtrate can be easily decanted, and the organic phase can be recycled as reaction solvent. Filtrate from the solvent aided hydrolysis requires a separation of hexane (or other solvent) from the reaction solvent before the organic phase can be considered for recycling.

The investigation which led to this improvement was directed particularly toward improving the process for synthesis of N-(4-anilinophenyl) methacrylamide (E) through reaction of methyl methacrylate with p-aminodiphenylamine in the presence of sodium methoxide.

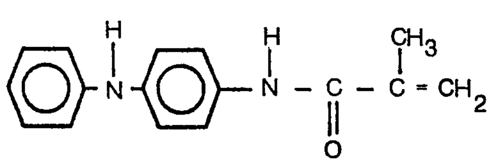

(E)

Preferred conditions for the in-situ hydrolysis of the intermediate sodium salt in this case are 21—24°C and a 1:1 volume ratio of water to the reaction mixture to which it is added.

The improvement of this invention will be further clarified by a consideration of the following examples, which are intended to be purely exemplary. The following definitions apply to terminology used in the data reported:

$$\%\ \text{Crude Yield} = \left(\frac{\text{wt crude prod sample}}{\text{wt slurry sample}}\right) \times \left(\frac{\text{tot slurry wt}}{\text{theoretical yld}}\right) \times 10C$$

"Slurry" is the reaction mixture after the first reaction (before addition of water). "Crude product" is the dried product after hydrolysis (no analysis for purity).

$$\text{Filtration Rate} = \frac{(\text{gms. product slurry}/545) \times 60(\frac{\text{min}}{\text{hr}})}{(0.0664\ \text{ft}^2) \times (\text{filtration time in min.})}$$

"Product Slurry" = 2 phase mixture obtained after hydrolysis. 454 = grams per pound.

Melting point is indicative of product purity, a range of 104—105°C. corresponding to almost pure product.

Also, in the data which follows, the term "standard hydrolysis" refers to the old method described in the background section, U.S. Patent No. 3,907,893.

Example I

Table 1 shows the data generated from various hydrolysis experiments utilizing the sodium salt slurry from a pilot plant product run of N-(4-anilinophenyl) methacrylamide. Most of the batches were made with an amount of water equal to the amount of reaction slurry, and the two phases were agitated with mold agitation (250 rpm, half-moon teflon agitator in one liter round bottom flask) at 21—24°C. for 30 minutes. Teflon is the registered trademark of E.I. Dupont de Nemours. The resulting product slurry was then filtered, washed with water, and the cake was dried in an oven at 60°C These conditions gave the best quality product with satisfactory yield. a few variations in temperature and amount of water were tried, but only adverse effects either in filtration or quality were noticed.

It was apparent from the very beginning that the solubilities of p-aminodiphenylamine, impurities, and the product in the mixed xylene solvent being used would play an important role in determining the right conditions for hydrolysis. Increasing the temperature increases the solubility of the p-aminodiphenylamine in the mixed xylenes, but it also increases the solubility of the product, resulting in less recoverably yield. Decreasing the temperature reverses this trend and the p-aminodiphenylamine or other impurities precipitate out affecting the quality of the product.

TABLE 1

| Laboratory Run # | Slurry gms | Water gms | Mixed Xylenes gms | Hexane gms | Hydrolysis Temp °C. | Filtration Rate kg/hr/m² | % Crude Yield | Comment |
|---|---|---|---|---|---|---|---|---|
| 88—1 | 655 | Standard hydrolysis | | | 21—24 | — (pH=12.5) | 66.8 | Slurry filtered and washed with mixed xylenes and hexane and then reslurried in water and filtered again |
| 89—4 | 500 | 500 | | 375 | 21 824 | | 77.0 | |
| 89—6 | 200 | 100 | 100 | 100 | 21—24 | 4857.5 | 74.4 | |
| 90—7 | 200 | 200 | 100 | 100 | 21 | 4384.9 | 74.4 | |
| 90—8 | 200 | 200 | | | 21—24 | 7773.6 | 54.7 | |
| 91—9 | 200 | 200 | | 150 | 21—24 | 5345.8 | 74.4 | |
| 91—10 | 200 | 200 | | 50 | 21 | 4374.3 | 63.4 | |
| 91—11 | 400 | Standard hydrolysis | | | 21—24 | 3466.2 | 73.3 | Hydrolyzed Product wouldn't filter at all at pH of 12.6 |
| 92—12 | 200 | 200 | 35 | — | 21—24 | 8453.7 | 54.7 | Mixed xylenes used for rinsing the flask. |
| 93—13 | 400 | 400 | 38 | — | 21—24 | 8143.2 | 54.7 | |
| 95—14 | 200 | 200 | 50 | — | 21—24 | 8743.6 | 50.3 | |
| 95—15 | 200 | 400 | 50 | — | 24 | — | — | |
| 54—4 | 200 | 200 | — | — | 40 | — | 43.75 | |
| 54.5 | 200 | 200 | — | — | 15 | — | 55.8 | |

Example II

The new hydrolysis technique has also been tried in pilot plant scale, using a 500 gallon reactor for the first reaction. Table 2 shows melting point and purity data on the final product of 9 pilot plant runs. After formation of the metallic salt in the first reaction, half of batch 38 was filtered, washed and hydrolyzed by the standard hydrolysis technique in two splits because of pilot plant equipment size limitations. The filter used in all the runs was a horizontal plate type pressure filter. Filter papers (grade 8A) were used in place of filter cloths to prevent blinding and improve filtration rate. Filtration, washing and nitrogen blowing through the filter cake were quite satisfactory compared to former runs using filter cloths. Filter cake resistance (i.e. pressure drop through the cake) was about 30 psig (207 kPa).

Since the removal of the filter cake from the filter would have meant the removal of plates and solvent exposure to the operator, hydrolysis in the filter by water washing was planned. This hydrolysis was quite slow. It took almost 12 hours to bring the ph of the effluent down to 9.5 from 12.6. It was desired to bring ph down to 7.0 to assure total absence of alkaline material from the cake. The water washing of the cake was stopped and the cake was air dried by blowing high pressure (90 psig, 620 kPa) air through the cake for one hour. The cake, when taken out of the filter, was quite wet, (38% solids) and had a strong odor of xylenes. As shown in Table 2, the final product melting point and purity were slightly lower than desired in both splits. The product from the second split has a dark gray color suspected to be due to the impurities left behind by solvent evaporation.

Due to the long time involved and the product quality, a decision was made to try in-situ hydrolysis on the remaining slurry from batch 38. The slurry was cooled to 24°C., and an amount of water equal to the slurry was added to the reactor. The water-organic mixture was then agitated for one hour and was recirculated through the filter for one hour. The filtrate was milky in color due to a suspected emulsion of xylene in water. The pressure drop through the cake was only 10 psig (68.9 kPa) even after one hour. The filtrate was then directed to a filtrate collection (decant) tank. The filter cake was blown with nitrogen and washed with water. The cake was then dried by air blowing for one hour and was removed from the filter. The cake was wet (48% solids) but was relatively free of solvent. The product, when dried, had a light green color and had a melting point of 104—105°C. The polymerization of this product was also quite satisfactory.

For comparison, the first half of Batch 38 required 32 hours to filter and hydrolyze; whereas, the second half required only 8 hours.

Batches 39 to 45 were finished in two splits, but each split was in-situ hydrolyzed as the third split from batch 38 above. At the end of the first reaction, half of the slurry (about 1400 lbs or 636Kg) was transferred from the 500 gallon (1895 liters) reactor to a 400 gallon (1516 liters) weight tank and was there with agitation and under a nitrogen blanket. The other half of the slurry was then in-situ hydrolyzed, filtered, water washed and the cake placed in a tray drier. The slurry from the 400 gallon tank was then transferred to the 500 gallon reactor and finished as above. Batch 46 was a half-size batch and was filtered in one split.

The filter cake wetness was reduced by properly positioning the plates in the filter to prevent the cake from being eroded by incoming slurry and water wash. Batches 41, 42, and 45 had 74%, 76%, and 64% solids respectively. The filter cake from Batch 42 was vacuum dried in a laboratory vacuum drier and the condensate collected. Material balance showed that the filter cake contained 76% solids, 20.4% water and 3.6% mixed xylenes.

Product quality of all the in-situ hydrolyzed product was excellent, except Batch 43 which had a faint purple cast to it and Batch 44 (split one) which had a dark gray color and had a low melting point. It is believed that these two cases were due to operator error in either hydrolysis (not enough water added) or filtration. Both batches were tested for polymerization and were found satisfactory.

TABLE 2

| Batch # | Split # | Yield % of Theoretical* | Melting Point +°C | Purity (by Gas Chromatography) |
|---|---|---|---|---|
| –38 | 1 | 60.5 | 102 | 86.3 |
| | 2 | | 101 | 85.1 |
| | 3 | | 104—5 | 96.7 |
| –39 | 1 | 60.5 | 104 | 95.01 |
| | 2 | | 104 | 96.38 |
| –40 | 1 | 60.5 | 105 | 94.96 |
| | 2 | | 104.5 | 96.23 |
| –41 | 1 | 53.5 | 105 | |
| | 2 | | 104 | 95.0 |
| –42 | 1 | 51.7 | 104 | 95.8 |
| | 2 | | 104 | 95.4 |
| –43 | 1 | 50.4 | 104 | 96.2 |
| | 2 | | 105 | 97.5 |
| –44 | 1 | 57.8 | 102 | 91.3 |
| | 2 | | 104 | 96.2 |
| –45 | 1 | 62.3 | 104 | 83.9 |
| | 2 | | 103 | 81.7 |
| –46 | 1 | 50.2 | 105 | 96.2 |

| | | |
|---|---|---|
| Batch Charge Quantities: | Mixed Xylenes | 2,160 lbs (980 kg) |
| | p-aminodiphenyl amine (PADPA) | 460 lbs (209 kg) |
| | Methyl methacrylate | 345 lbs (161 kg) |
| | Sodium methoxide | 150 lbs ( 68 kg) |

*Theoretical yield based on p-aminodiphenylamine

$$\text{charged} = \frac{460 \text{ lbs}}{184 \text{ lbs-lb mole PADPA}} \times \frac{252 \text{ lbs}}{\text{lb mole Product}} = 630 \text{ lbs } (286 \text{ kg})$$

**Expected yield based on 60% conversion of p-aminodiphenyl amine = 378 lbs (171 kg)

+Melting Point of N-(4-anilinophenyl) methacrylamide standard (recrystallized, 99.9%) = 106°C.

Batches 38, 39 and 40 had identical yield of 60.5%, but Batches 41, 42, 43 and 46 had substantially lower yield. It is believed that lower yields were a result of handling losses and losses in the filtrate due to holes in the filter paper. When the filter cake from the first split of Batch 43 was removed from the filter, there were quite a few holes in the paper on the top plate, and there was no cake built-up in those areas. A substantial amount of product was found in the filtrate tank. It is believed that paper was accidentally cut while assembling the filter. Batch 46 was reacted with the normal amount mixed xylenes but with half the amount of the reactants (to finish off sodium methoxide on hand). The low yield from this batch is attributed to the lower concentration of reactants.

It is believed that a better separation of the hydrolyzed product can be obtained by use of a centrifuge rather than a filter.

Other embodiments of this invention will be apparent to those skilled in the art from a consideration of this specification or practice of the invention disclosed herein. It is intended that the specification and examples be considered as exemplary only, with the true scope and spirit of the invention being indicated by the following claims.

**Claims**

1. An improved process for the synthesizing of an antioxidant amide comprising:

I. reacting, in an aromatic solvent, an aliphatic or aromatic ester, either saturated or unsaturated, having the formula:

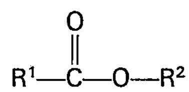

(A)

with an aromatic amine having the general formula:

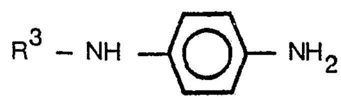

(B)

in the presence of (C) a base selected from the group consisting of alkali metal amides and alkali metal alkoxides to yield an intermediate metallic salt:

II. hydrolyzing the salt in situ by mixing the reaction product from step I with water at a temperature at which most of the reaction product is suspended in the water phase and most of the unreacted aromatic amine is dissolved in the reaction solvent phase to form an antioxidant amide of the general formula:

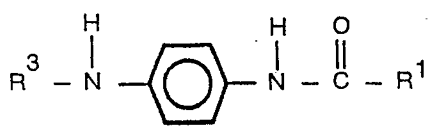

(D)

wherein $R^1$ is selected from the group consisting of alkyl radicals having from 1 to 20 carbon atoms, aryl radicals having from 6 to 12 carbon atoms, cycloalkyl radicals having from 5 to 12 carbon atoms, alkenyl radicals having from 2 to 10 carbon atoms, $R^2$ is selected from the group consisting of alkyl radicals having from 1 to 10 carbon atoms, and alkenyl radicals having from 2 to 10 carbon atoms, and wherein $R^3$ is selected from the group consisting of alkyl radicals having from 1 to 20 carbon atoms, cycloalkyl radicals having from 5 to 12 carbon atoms, aryl radicals having from 6 to 12 carbon atoms, and aralkyl radicals having from 7 to 13 carbon atoms.

2. The process as recited in Claim 1 which further comprises removing the antioxidant amide from the reaction mixture after hydrolysis and drying the antioxidant amide.

3. The process as recited in Claim 2 wherein the ester (A) is methyl methacrylate, the aromatic amine (B) is para-aminodiphenylamine, and the base (C) is sodium methoxide.

4. The process as recited in Claim 3 wherein the in-situ hydrolysis is carried out at 21—24°C. and with a 1:1 volume ratio of water to the reaction mixture before hydrolysis.

**Revendications**

1. Procédé perfectionné pour la synthèse d'un amide antioxydant, ce procédé comprenant les étapes consistant à:

I. faire réagir, dans un solvant aromatique, un ester aliphatique ou aromatique, saturé ou insaturé et répondant à la formule:

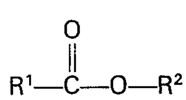

(A)

avec une amine aromatique répondant à la formule générale:

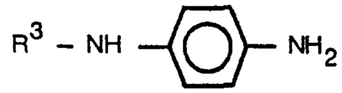 (B)

en présence de (C) une base choisie parmi le groupe comprenant les amidures de métaux alcalins et les alcoxydes de métaux alcalins pour obtenir en sel métallique intermédiaire;

II. hydrolyser le sel in situ en mélangeant le produit réactionnel de l'étape I avec de l'eau à une température à laquelle la majeure partie du produit réactionnel est en suspension dans la phase aqueuse, tandis qui la majeure partie de l'amine aromatique n'ayant pas réagi est dissoute dans la phase de solvant réactionnel pour former un amide antioxydant de formule générale:

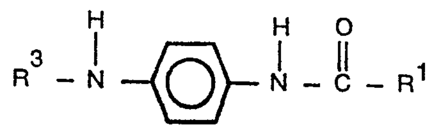 (D)

dans laquelle

$R^1$ est choisi parmi le groupe comprenant les groupes alkyle contenant 1 à 20 atomes de carbone, les groupes aryle contenant 6 à 12 atomes de carbone, les groupes cycloalkyle contenant 5 à 12 atomes de carbone, les groupes alcényle contenant 2 à 10 atomes de carbone; $R^2$ est choisi parmi le groupe comprenant les groupes alkyle contenant 1 a 10 atomes de carbone et les groupes alcényle contenant 2 à 10 atomes de carbone, tandis que $R^3$ est choisi parmi le groupe comprenant les groupes alkyle contenant 1 à 20 atomes de carbone, les groupes cycloalkyle contenant 5 à 12 atomes de carbone, les groupes aryle contenant 6 à 12 atomes de carbone et les groupes aralkyle contenant 7 à 13 atomes de carbone.

2. Procédé suivant la revendication 1, caractérisé en ce qu'il consiste également à éliminer l'amide antioxydant du mélange réactionnel après hydrolyse et à sécher cet amide antioxydant.

3. Procédé suivant la revendication 2, caractérisé en ce que l'ester (A) est le méthacrylate de méthyle, l'amine aromatique (B) est la para-aminodiphénylamine et la base (C) est le méthoxyde de sodium.

4. Procédé suivant la revendication 3, caractérisé en ce qu'on effectue l'hydrolyse in situ à une température de 21—24°C et à un rapport en volume de 1:1 entre l'eau et le mélange réactionnel avant l'hydrolyse.

**Patentansprüche**

1. Verfahren zur Synthetisierung eines als Antioxidationsmittel eingesetzten Amids, dadurch gekennzeichnet, daß

I. in einem aromatischen Lösungsmittel ein aliphatischer oder aromatischer gesättigter oder ungesättigter Ester der Formel

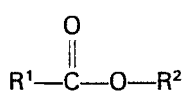 (A)

mit einem aromatischen Amin der allgemeinen Formel

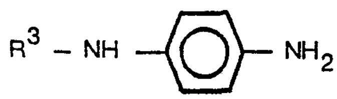 (B)

in Gegenwart von (C) einer Base, ausgewählt aus der Gruppe, die aus Alkalimetallamiden und Alkalimetallalkoxiden besteht, zur Gewinnung eines als Zwischenprodukt anfallenden metallischen Salzes umgesetzt wird,

II. das Salz in situ durch Vermischen des Reaktionsproduktes aus der Stufe I mit Wasser bei einer Temperatur, bei der die Hauptmenge des Reaktionsproduktes in der Wasserphase suspendiert und die Hauptmenge des nichtumgesetzten aromatischen Amids in der Reaktionslösungsmittelphase aufgelöst wird, unter Bildung eines als Antioxidationsmittel wirkenden Amids der allgemeinen Formel

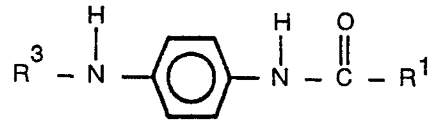

(D)

hydrolysiert wird, wobei $R^1$ aus der Gruppe ausgewählt ist, die aus Alkylresten mit 1 bis 20 Kohlenstoffatomen, Arylresten mit 6 bis 12 Kohlenstoffatomen, Cycloalkylresten mit 5 bis 12 Kohlenstoffatomen und Alkenylresten mit 2 bis 10 Kohlenstoffatomen besteht, $R^2$ aus der Gruppe ausgewählt ist, die aus Alkylresten mit 1 bis 10 Kohlenstoffatomen und Alkenylresten mit 2 bis 10 Kohlenstoffatomen besteht, und $R^3$ aus der Gruppe ausgewählt ist, die aus Alkylresten mit 1 bis 20 Kohlenstoffatomen, Cycloalkylresten mit 5 bis 12 Kohlenstoffatomen, Arylresten mit 6 bis 12 Kohlenstoffatomen sowie Aralkylresten mit 7 bis 13 Kohlenstoffatomen besteht.

2. Verfahren nach Anspruch 1, dadurch gekennzeichnet, daß das als Antioxidationsmittel verwendbare Amid aus der Reaktionsmischung nach der Hydrolyse entfernt und getrocknet wird.

3. Verfahren nach Anspruch 2, dadurch gekennzeichnet, daß der verwendete Ester (A) aus Methylmethacrylat, das aromatische Amin (B) aus p-Aminodiphenylamin und die Base (C) aus Natriummethoxid besteht.

4. Verfahren nach Anspruch 3, dadurch gekennzeichnet, daß die in situ-Hydrolyse bei 21 bis 24°C unter Einhaltung eines Volumenverhältnisses von Wasser zu der Reaktionsmischung von 1:1 vor der Hydrolyse durchgeführt wird.